Europäisches Patentamt

European Patent Office

Office européen des brevets

(11) Publication number: **0 105 747**
**A1**

# (12) EUROPEAN PATENT APPLICATION

(21) Application number: **83305938.9**

(22) Date of filing: **30.09.83**

(51) Int. Cl.³: **C 12 Q 1/30**
**G 01 N 33/52**

(30) Priority: **06.10.82 GB 8228524**

(43) Date of publication of application:
**18.04.84 Bulletin 84/16**

(84) Designated Contracting States:
**AT BE CH DE FR GB IT LI LU NL SE**

(71) Applicant: **Venturecare Limited**
**Janton House Moor Lane Bagby**
**Thirsk North Yorkshire YO7 2PN(GB)**

(72) Inventor: **Lewis, Ian Hartley**
**1 Moor Lane Bagby**
**Thirsk North Yorkshire Y07 2PN(GB)**

(74) Representative: **Orr, William McLean et al,**
**Haseltine Lake & Co Hazlitt House 28 Southampton**
**Buildings Chancery Lane**
**London WC2A 1AT(GB)**

(54) Hygiene-testing.

(57) There is disclosed a hygiene-testing kit for use in hygiene-testing a food-contacting surface (11) by responding to the presence of catalase on the surface. A coloured testing sample is derived by mixing a stable solution of concentrated hydrogen peroxide (B1) with a solution of distilled water containing a food approved dye (A2) which has no spontaneous reaction with hydrogen peroxide. A coloured droplet of the testing sample is applied to a portion of the food-contacting surface (11), and the wetted portion of the surface is then observed so as to detect the presence or the absence of gas bubbles being evolved in the coloured droplets (12, 13, 14). Alternatively, the food-contacting surface is tested by taking a sample using a swab (17) and then the sample is exposed to the action of the coloured testing sample, and observation made as to the presence or absence of gas bubbles being evolved. Evolution of gas bubbles gives an immediate indication of unhygienic conditions prevailing, and this detection of the gas bubbles is readily apparent since the gas bubbles (oxygen) are colourless, and can be seen readily against the coloured background provided by the testing sample.

FIG. 1

-1-

## Hygiene-testing

This invention relates to a method and apparatus for the hygiene-testing of a food-contacting surface. The term "food-contacting surface" herein is intended to refer to working and other surfaces which come into contact with, or are liable to come into contact with foodstuffs, either in the raw or processed state, in the food handling and processing industries.

Examples of food-contacting surfaces are as follows:-

1. Working surfaces,
2. Knives and other utensils,
3. Protective clothing ,
4. Containers,
5. Machine parts in food-processing equipment,
6. Live animal food troughs, handling and housing equipment and vehicles,
7. Horticultural medium handling and housing premises and equipment.

In the food handling and processing industries, it is essential that very careful control be exercised to maintain necessary standards of hygiene. Although stringent in requirements, current test procedures require very careful testing to be carried out, which is time-consuming in obtaining samples at the point of testing, and also usually involves a time delay in obtaining the test results, in that these are only obtained after taking the samples to a laboratory for further tests.

There are evident disadvantages to the existing procedures, in that either the equipment being tested must lie idle until hygiene-clearance is given, or else there is the risk of unhygienic operation in the interim period between the taking of the samples and issue of the test results.

The present invention has been developed with a view to enable hygiene-testing of a food contacting surface to be carried out in simple manner not requiring more than ordinary skills, and for immediate indication to be given of hygienically safe or unsafe conditions.

The invention is based on the fact that dilute hydrogen peroxide spontaneously gives-off oxygen in the presence of catalase, which is an enzyme which is present in raw food residues and many food-spoilage micro-organisms.

According to one aspect the invention provides a method of hygiene-testing a food-contacting surface, as herein defined, by responding to the presence of catalase on the surface, characterised by the steps of forming a coloured testing sample by mixing a stable solution of concentrated hydrogen peroxide with a solution of distilled water containing a food-approved dye which has no spontaneous reaction with hydrogen peroxide, applying a coloured droplet of the testing sample to a portion of the food-contacting surface, and observing the wetted portion of the surface so as to detect the presence or the absence of gassbubbles being evolved in the coloured droplet.

When the surface to be tested is not readily accessible, or cannot readily receive a droplet of the testing solution, the invention provides a further method of hygiene-testing a food-contacting surface, as herein defined, by responding to the presence of catalase on the surface, characterised by the steps of forming a colour testing sample by mixing a stable solution of concentrated hydrogen peroxide with a solution of distilled water containing a food-approved dye which has no spontaneous reaction with hydrogen peroxide, obtaining a sample from a portion of the food-contacting surface with a swab, exposing the swab with the sample to the action of the coloured testing sample, and observing the swab with the sample thereon so as to detect the presence or the absence of gas bubbles being evolved.

The swab with the sample thereon may be exposed to the action of the coloured testing sample by insertion of the swab into a container holding the coloured testing sample, and the observation of the evolution of gas bubbles (colourless) can readily be detected against the surrounding coloured background provided by the coloured testing sample in the container.

Alternatively, the swab with the sample thereon may be exposed to the action of the coloured testing sample by application of one or more droplets of the coloured testing sample e.g. from a pipette, which then fall onto a horizontal testing plate at which the observation for evolution of gas bubbles can then readily be carried out.

If the food-contacting surface which is being tested is wet, then the swab (which will be a sterilized swab) can be applied immediately to selected test regions of the surface. However, if the surface is relatively dry, then the swab will first of all be moistened by distilled water.

Preferably, the method uses a hygiene-testing kit which comprises separate supplies of the concentrated hydrogen peroxide, and of the distilled water/dye solution. The kit can then have a long "shelf life", in that concentrated hydrogen peroxide is stable and does not deteriorate, whereas dilute hydrogen peroxide deteriorates with time. Thus, the user can prepare the mixture from the two supplies on site so that a "fresh" testing mixture (including diluted hydrogen peroxide) is always available.

The presence of the dye gives colouring to the testing sample, thereby rendering ready detection of any gas bubbles which may be evolved. As indicated above, the dye should be a food-approved dye i.e. a dye which is approved for use in the food-handling and processing industries. Also, the dye should not involve any spontaneous and premature generation of oxygen when the dye solution is mixed with the concentrated hydrogen peroxide.

Evidently, the supplies of the two constituents of the testing mixture could be provided in many different ways. For example, predetermined quantities could be stored in individual containers, which are then mixed immediately before testing takes place. Alternatively, predetermined quantities could be held in separate chambers within a single container, with a frangible connection between the chambers. Thereafter, to prepare a supply of the testing mixture, it is only necessary to break the connection between the two chambers, and allow the contents of the chambers to mix, prior to the application of a droplet of the mixture to a surface to be tested.

In another arrangement, the kit may have separate bulk supplies of the concentrated hydrogen peroxide and the dye solution, and a measuring device, such as a pipette is provided, to enable predetermined amounts to be drawn-off and mixed.

It is preferred that the hydrogen peroxide/dye solution should dilute the hydrogen peroxide so as to have a dilution in the range 3 vols to 200 vols.

Conveniently, the testing kit is readily portable, in that the contents of the kit are stored in an attache case or other similar carrier.

The invention may also be applied in the automatic indication of unhygienic conditions.

The invention will now be described in more detail, by way of example only, with reference to the accompanying drawings in which:-

Figure 1 is a schematic view of a portable hygiene- testing kit for use in carrying out a method according to the invention;

Figure 2 is a schematic illustration of the use of the kit in the hygiene-testing of a food-contacting surface; and

Figures 3a to 3d illustrate an alternative mode of use of the hygiene testing kit.

Referring now to the drawings, a hygiene-testing kit is provided for hygiene-testing a food-contacting surface by responding to the presence of catalase on the surface. The kit is portable, and comprises a number of reagents arranged in an attache case 10 or similar carrier. The case 10 has suitable compartments to accommodate the various components of the kit, which comprises a supply A1 of distilled water for damping swabs, a supply A2 of distilled water containing a food-approved dye, a supply B1 of a stable solution of concentrated hydrogen peroxide, and a supply C of iodine solution for carrying out a starch test. A measuring cylinder or other container X is provided, into which measured quantities of supply A2 and supply B1 are inserted in order to obtain a coloured testing sample. The supplies A2 and B1 are mixed together, and the food-approved dye contained in supply A2 is of such a type as to have no spontaneous reaction with hydrogen peroxide.

Concentrated hydrogen peroxide is a stable solution, and therefore can be readily kept in concentrated form in the supply B1 without deterioration. It is only upon dilution that hydrogen peroxide becomes less stable, so that a coloured testing sample is obtained immediately prior to a testing procedure.

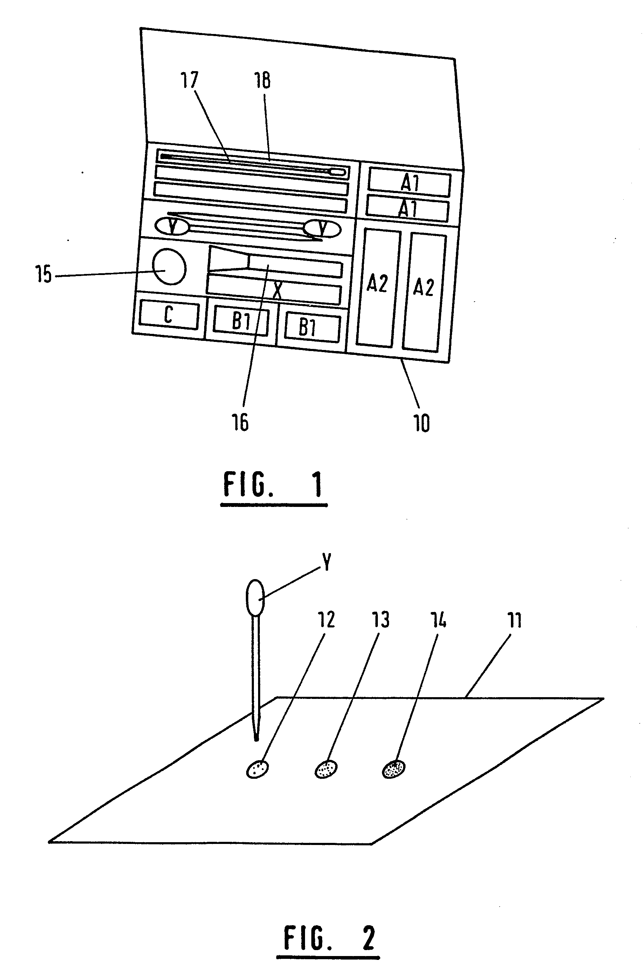

There are two modes of use of the hygiene-testing kit, and the first mode is shown schematically in Figure 2. A typical generally horizontal food-contacting surface 11 is shown in Figure 2, and a coloured droplet of the testing sample is applied by means of pipette Y to one or more selected portions of the food-contacting surface. Upon application of a droplet to a selected area of the surface 11, the wetted portion of the surface is then observed so as to detect the presence or the absence of gas bubbles being evolved in the coloured droplets. Hydrogen peroxide is reduced to water and oxygen by the catalase enzymes present in raw food residues and many food spoilage micro-organisms. This reaction takes place with the catalase enzymes acting as a catalyst, within broad parameters of pressure and temperature. Therefore, the absence of any evolution of gas bubbles is a clear indication of hygienic conditions. Depending upon the degree of gas evolution, this will give a clear indication of the extent to which the surface 11 is unhygienic. Figure 2 shows, by way of example only, a coloured droplet 12 showing only slight evolution of gas, a coloured droplet 13 showing moderate evolution, and a droplet 14 showing a high evolution of gas, therefore very unhygienic conditions. The evolution of the gas bubbles (which are colourless) can readily be observed against the coloured background provided by the coloured droplets in which they are evolved.

A magnifying glass 15 is provided in the kit, and also a pen torch 16, to facilitate observation of gas evolution, especially when there is only very slight gas evolution.

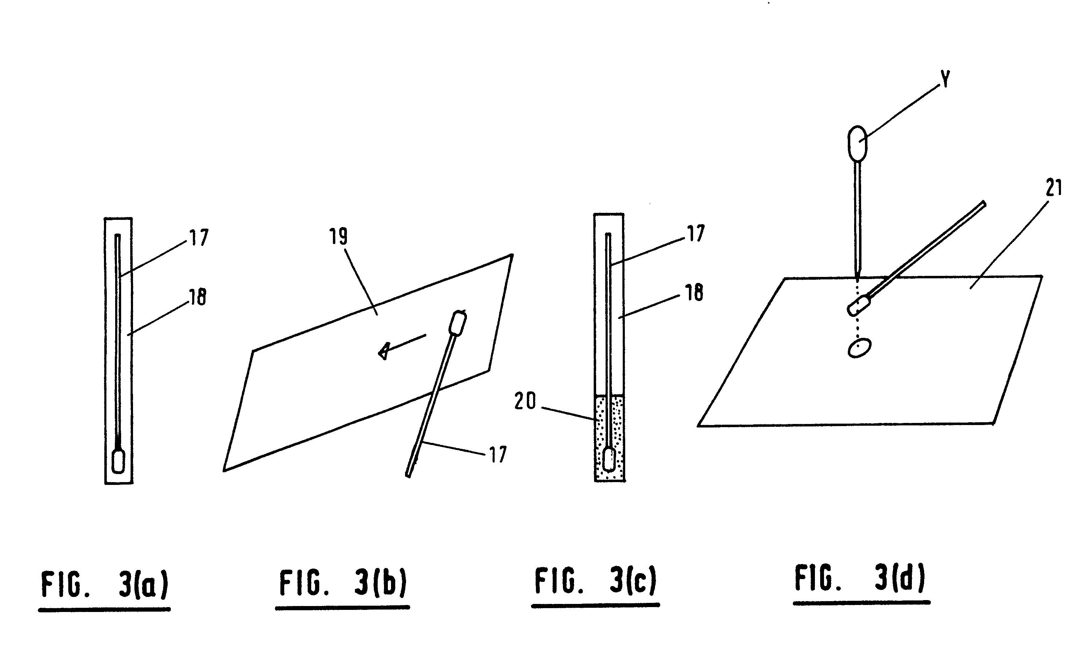

The mode of use shown in Figure 2 is suitable for situations in which the food-contacting surface is horizontal or generally horizontal. However, when the food-contacting surface is substantially inclined to the horizontal, or if the surface to be tested is not readily accessible, or cannot readily receive a droplet from the pipette Y, the alternative mode of use shown in Figure 3a to 3b may be used.  The kit includes, for this alternative use, a series of sterile swabs 17 packed in sterile conditions in glass containers 18.  A swab 17 is applied to a selected area or areas of a food contacting surface which by way of example only, is shown as an inclined surface 19 in Figure 3b.  If the surface 19 is dry, then the swab 17 is wetted by application of distilled water from supply A1.  Once a sample has been collectedon swab 17, the swab is then exposed to the action of the coloured testing sample.  This may be achieved in two ways.  The first way is shown in shown in Figure 3c in which the swab 17 with the sample is re-inserted in glass tube 18 which has  been previously filled with a measure 20 of the coloured testing sample.  When catalase is present on the sample, there will be a ready indication of gas evolution, set against the coloured background provided by the surrounding medium 20.

Alternatively, the swab 17 with the sample thereon may have a droplet of the testing sample applied thereto by the pipette X, in the manner shown in Figure 3d.  The droplet then falls onto a horizontal test plate 21, and observation is carried out for any evolution of gas, as described above, to test for hygienic conditions on the surface 19.

If desired, the reagents in the testing kit (other than the already coloured supply A2), may be colour coded with distinctive colourings by application of suitable food-approved dyes which do not have any adverse reaction on the spontaneous generation of oxygen when hydrogen peroxide is exposed to the presence of catalase.  Thus, the supply B1 may be pre-mixed with a dye sold under the trade name Edicol Supra Tartrazine NS.

A particularly suitable dye used in supply A2 is Amaranth 1508 made by D.F. Anstead.   The formation of a coloured testing sample from supplies A2 and B1 then provide a red colouring which is particulary suitable as a background to the observation of the evolution of colourless oxygen therein.

Other dyes used in supply A2 may be one of food-approved dyes made by McCormick. as follows:

1.   Propylene Glycol
2.   Tartrazine E102
3.   Amaranth E123
4.   Green S142.

Alternatively, good performance can be obtained with a dye made by I.C.I. and sold under the name Edicol Suprablue EGS.

In the use of the kit, when unhygienic conditions are present, gas bubbles will normally be evolved within about 45 seconds.  If no bubbling occurs, it is safe to assume that the area being tested is free from contamination.  However, if bubbling occurs, the area being tested must be .thoroughly cleaned and then rechecked by further testing, before food can be allowed to come into contact with it.

The hygiene-testing kit may also be used to detect contamination related to starch-based materials, when this is suspected i.e. when flour or bakery waste etc. is present.  In this case, the liquid reagent C1 is used to carry out the test, by being applied to a sterile swab 17, and then wiped over the area to be checked.  Areas with starch based contamination will show on the tip of the swab by a change in colour from brown to blue.

The portable hygiene-testing kit shown in Figure 1 is schematic only, and may be  substantially enlarged, as to size and contents.  In addition, modifications are possible.  For example, one or more of the supplies may be provided in pre-measured capsules or liquid containers.  Also, the supply B1 may be replaced by an automatic droplet ejection instrument, which is self-charging and meters direct from liquid reagents capsules (two stage).

The range of dilution of the supply A2 and supply B1 may be varied to suit the requirements for sensitivity, and the dye concentration also will be varied to suit requirements.

Although not shown, if desired the case 10 may be provided with a removable stage which can be transferred to a specific testing area, while retaining a recording facility in the case and preventing breakage or the entire unit becoming damaged or soiled.

Claims:

1. A method of hygiene-testing a food-contacting surface, as herein defined, by responding to the presence of catalase on the surface, characterised by the steps of forming a coloured testing sample by mixing a stable solution of concentrated hydrogen peroxide with a solution of distilled water containing a food-approved dye which has no spontaneous reaction with hydrogen peroxide, applying a coloured droplet of the testing sample to a portion of the food-contacting surface, and observing the wetted portion of the surface so as to detect the presence or the absence of gas bubbles being evolved in the coloured droplet.

2. A method of hygiene-testing a food-contacting surface, as herein defined, by responding to the presence of catalase on the surface, characterised by the steps of forming a coloured testing sample by mixing a stable solution of concentrated hydrogen peroxide with a solution of distilled water containing a food-approved dye which has no spontaneous reaction with hydrogen peroxide, obtaining a sample from a portion of the food-contacting surface with a swab, exposing the swab with the sample to the action of the coloured testing sample, and observing the swab with the sample thereon so as to detect the presence or the absence of gas bubbles being evolved.

3. A method according to claim 2, characterised in that the swab with the sample is inserted into a container holding the coloured testing sample, and the swab with the sample is observed within the container against the background of the surrounding coloured testing sample.

4. A method according to claim 2, characterised in that the swab with the sample is exposed to the action of the coloured testing sample by applying droplets of the testing sample to the swab, to fall on a horizonal test plate at which observation of gas evolution is carried out.

5. A method according to any one of the preceding claims, characterised in that the food-approved dye in the distilled water solution is one or more of the dyes sold under the trade names Propylene Glycol, Tartrazine E 102, Amaranth E 123, and Green S142 obtainable from McCormick, Edicol Suprablue EGS made by ICI, or Amaranth 1508 made by D.F. Anstead Limited.

6. A hygiene-testing kit for carrying out a method according to claim 1, comprising a supply of concentrated hydrogen peroxide (B1), a supply of distilled water with a food-approved dye (A2), means (X) for mixing the solution of concentrated hydrogen peroxide (B1) with the solution of distilled water with food-approved dye (A2) to form a testing sample, and means (Y) for applying a droplet of the testing sample to a portion of a food-contacting surface whose hygiene is to be tested.

7. A hygiene-testing kit according to claim 6 for carrying out a method according to claim 1, characterised by one or more sterile swabs.

8. A hygiene testing kit according to claims 6 or 7, characterised by a supply of iodine solution for carrying out a starch test.

FIG. 1

FIG. 2

FIG. 3(a)  FIG. 3(b)  FIG. 3(c)  FIG. 3(d)

0105747

European Patent Office

**EUROPEAN SEARCH REPORT**

| | DOCUMENTS CONSIDERED TO BE RELEVANT | | EP 83305938.9 |
|---|---|---|---|

| Category | Citation of document with indication, where appropriate, of relevant passages | Relevant to claim | CLASSIFICATION OF THE APPLICATION (Int. Cl. ³) |
|---|---|---|---|
| A | GB - A - 1 254 417 (BOEHRINGER MANNHEIM G.M.B.H.)<br><br>* Claim 1 *<br><br>-- | 1,2,5 | C 12 Q   1/30<br><br>G 01 N 33/52 |
| X | DR. OTTO-ALBRECHT NEUMÜLLER "Römpps Chemie-Lexikon", 7. Auflage, Band 3, 1973<br><br>FRANCKH'SCHE VERLAGSHANDLUNG, Stuttgart<br>Seiten 1669, 1728<br><br>-- | 1,8 | |
| A | US - A - 4 065 357 (GROVES)<br><br>* Abstract *<br><br>---- | 1-3 | |

| | TECHNICAL FIELDS SEARCHED (Int. Cl. ³) |
|---|---|
| | C 12 Q<br><br>G 01 N 33/00 |

The present search report has been drawn up for all claims

| Place of search | Date of completion of the search | Examiner |
|---|---|---|
| VIENNA | 22-12-1983 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82